Europäisches Patentamt
European Patent Office
Office européen des brevets

(19) (11) Publication number: **0 066 254 B1**

(12) # EUROPEAN PATENT SPECIFICATION

(45) Date of publication of patent specification: **04.02.87**

(21) Application number: **82104603.4**

(22) Date of filing: **26.05.82**

(51) Int. Cl.[4]: **C 07 C 153/07,** C 07 C 155/02, C 07 C 149/30, C 07 C 147/06, C 07 C 149/23, C 07 C 153/09, C 07 C 161/03, C 07 C 161/05, A 61 K 31/22, A 61 K 31/255, A 61 K 31/265

(54) **Mercapto amino acids, process for their preparation and pharmaceutical compositions containing them.**

(30) Priority: **01.06.81 US 269166**

(43) Date of publication of application: **08.12.82 Bulletin 82/49**

(45) Publication of the grant of the patent: **04.02.87 Bulletin 87/06**

(84) Designated Contracting States: **BE CH DE FR GB IT LI NL SE**

(56) References cited:
**EP-A-0 001 989**
**EP-A-0 014 167**
**EP-A-0 034 205**
**GB-A-2 054 586**
**GB-A-2 077 719**

(73) Proprietor: **USV PHARMACEUTICAL CORPORATION**
**1 Scarsdale Road**
**Tuckahoe New York (US)**

(72) Inventor: **Youssefyeh, Raymond D.**
**435 Martling Avenue**
**Tarrytown, NY (US)**
Inventor: **Jones, Howard**
**5 Mountain Lane**
**Holmdel, NJ (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath**
**Maximilianstrasse 58**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

The present invention relates to mercapto amino acids of the formula I

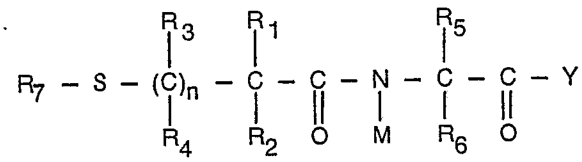

(I)

wherein

$R_1$, $R_2$, $R_3$, $R_4$, $R_5$, and $R_6$ are hydrogen, alkyl, alkenyl, alkynyl, aryl, aralkyl and cycloalkyl and may be the same or different,

n is an integer from 0 to 4 inclusive,

M is alkenyl, alkynyl, cycloalkyl, cycloalkylalkyl, polycycloalkyl, polycycloalkylalkyl, aryl, aralkyl, heterocyclic, heterocyclicalkyl, fused arylcycloalkyl, fused arylcycloalkylalkyl, fused heterocycliccycloalkyl, fused heterocycliccycloalkylalkyl, alkoxyalkyl, alkylmercapto, alkylaminoalkyl or dialkylaminoalkyl,

Y is hydroxy, alkoxy, amino, or substituted amino, alkanoylamino, aryloxy, aminoalkoxy or hydroxyalkoxy, and

$R_7$ is cycloalkyl, cycloalkylalkyl, heterocyclic, heterocyclicalkyl,

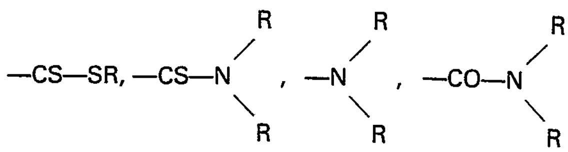

$—CH_2CO_2R$, $—CHM—CO_2R$, $—CH_2—CO—SR$, $—CO—NH—CH_2CO_2R$

or 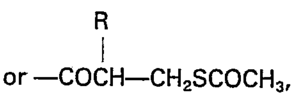, $—CO—CO_2R$,

wherein R is hydrogen, alkyl or aryl,

and salts thereof, especially pharmaceutically acceptable salts such as the alkali metal, alkaline earth metal, and amine salts, with the proviso that when $R_7$ is

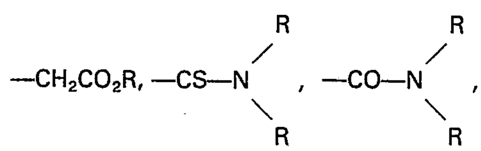

$—CO—CO_2R$, —CS—SR or cycloalkyl, M is not aryl.

These mercapto amino acids possess antihypertensive and angiotensin converting enzyme inhibitory activity. GB—A—2054586 discloses antihypertensive amides of the structure

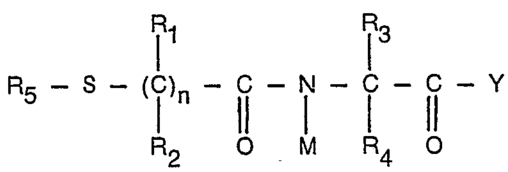

wherein

$R_1$, $R_2$, $R_3$, and $R_4$ are hydrogen, alkyl, alkenyl, alkynyl, phenyl-alkyl and cycloalkyl, and may be the same or different;

n is an integer from 1 to 5 inclusive;

M is alkenyl, alkynyl, cycloalkyl, aryl, heterocyclic, including both heteroaryl and heterocycloalkyl, hydroxyalkyl, mercaptoalkyl, aminoalkyl, alkoxyalkyl, alkylthioalkyl, alkylaminoalkyl or dialkylaminoalkyl;

Y is hydroxy, alkoxy, amino, alkanoylamino, aryloxy, aminoalkoxy, alkoxyalkoxy or hydroxyalkoxy; and

$R_5$ is hydrogen, alkyl, alkenyl, alkynyl, aryl, cyano, aralkyl, alkanoyl, arylalkanoyl, carboxyalkanoyl, hydroxyalkanoyl, aminoalkanoyl, amidino, carbalkoxy or ZS or

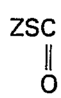

wherein Z is hydrogen, alkyl, hydroxyalkyl or the radical

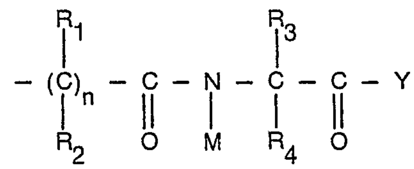

wherein

$R_1$, $R_2$, $R_3$, $R_4$, n, M and Y are as previously described herein;

and salts thereof.

GB—A—2077719 discloses compounds of the formula

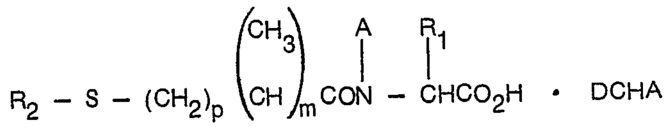

wherein

$R_1$ is hydrogen or a lower alkyl group,

$R_2$ is hydrogen, acetyl or benzoyl,

A is cyclopentyl, cyclohexyl or cycloheptyl,

m is 0 or 1

p is 1 or 2, and

DCHA is dicyclohexylamine or nil,

and their salts, which have activity in inhibiting angiotensin 1 converting enzyme.

In the compounds of the present invention the alkyl groups per se and the alkyl moiety in aralkyl, cycloalkylalkyl, polycycloalkylalkyl, heterocyclicalkyl and the like, and, in alkoxy, alkylmercapto, alkanoyl, carbalkoxy, and alkylamino, may be straight-chained or branched and are preferably lower alkyl groups containing from 1 to 6 carbons. Such groups include methyl, ethyl, propyl, isopropyl, butyl, isobutyl, amyl, iso-amyl or hexyl.

The alkenyl and alkynyl groups may also be branched or straight-chained and contain from 2 to 6 carbon atoms. Such groups include vinyl, ethynyl, propenyl, allyl or isopropenyl.

The M cycloalkyl, polycycloalkyl, aryl, heterocyclic, arylalkyl, fused arylcycloalkyl groups and the like contain from 3 to 16 carbon atoms and may be substituted with substituents such as lower alkyl, alkenyl, alkynyl, hydroxy, mercapto, amino, alkoxy, alkylmercapto, alkylamino, and halo. They include such radicals as cyclopropyl, cyclobutyl, cyclopentyl, cyclohexyl, cycloheptyl, adamantyl, norbornyl, phenyl, tolyl, benzyl, phenethyl, dimethoxyphenyl, hydroxybenzyl, indanyl, naphthyl, tetrahydronaphthyl, decahydronapthyl, pyridyl, quinolyl, pyrrolidyl, pyrrolyl, morphilinyl, furyl, furfuryl, tetrahydrofurfuryl, benzimidazolyl, thienyl or imidazolyl.

The $R_7$ heterocyclic groups include the heterocyclic groups listed for M above as well as heterocyclicalkyl, and heteroclic groups can be substituted with alkyl, alkenyl, alkynyl, hydroxy, hydroxyalkyl, alkoxy, halogen, mercapto, alkylmercapto, amino, aminoalkyl, alkylamino, nitro, cyano, carboxy, carbalkoxy, carboamido, sulfonyl, sulfonamido, trifluoromethyl, and methylenedioxy.

The aryl groups in M also include substituted aryl wherein the substituent is alkyl, alkenyl, alkynyl, hydroxyalkyl, alkoxy, halogen, mercapto, alkylmercapto, amino, aminoalkyl, alkylamino, nitro, cyano, carboxy, carbalkoxy, carboxamido, sulfonyl, sulfonamido, trifluoromethyl and methylenedioxy.

The preferred compounds are those wherein M is cycloalkyl having 3 to 7 carbon atoms, $R_1$, $R_3$, $R_4$, $R_5$ and $R_6$ are hydrogen, $R_2$ is hydrogen or lower alkyl, preferably methyl, $R_7$ is $COCO_2R$, $CON(R)_2$, $CH_2CO_2R$, or $COCH(CH_3)CH_2SCOCH_3$, n is 0 or 1, and Y is hydroxy, wherein R is hydrogen, lower alkyl or phenyl.

The compounds of the present invention can be prepared from a compound of formula II

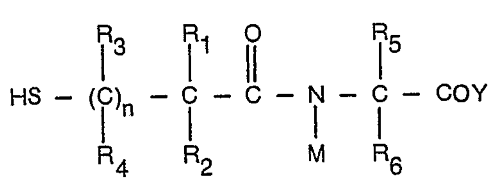

(II)

by replacement of the hydrogen or the SH group with a substituent representative of $R_7$ by usual replacement reactions, e.g., alkyl group by ether formation.

Alternatively, a compound of formula III

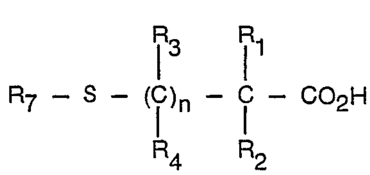

(III)

can be reacted under amide-forming conditions with a compound of formula IV

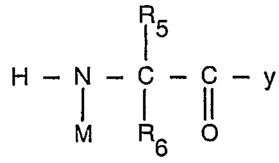

(IV)

to form a corresponding amide of formula I.

A further preparative method involves reaction of a compound of formula V

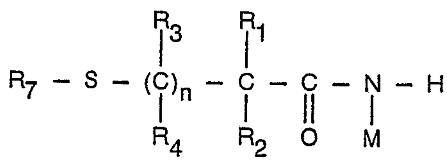

(V)

with an alpha haloacid of formula VI

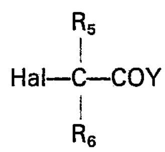

(VI)

with elimination of hydrogen halide to form corresponding compounds of formula I.

The intermediate compounds of formula II can be prepared by hydrolysis of the corresponding acylated mercapto compounds of formula VII

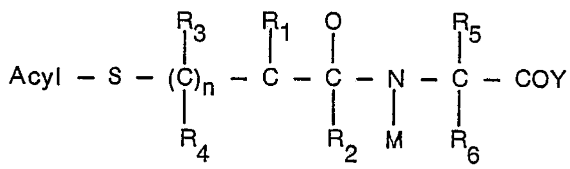

(VII)

which can be prepared by reaction of a compound of formula VIII

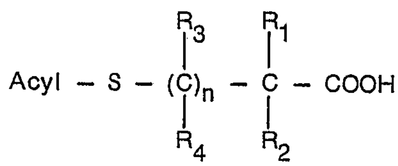

(VIII)

under amide-forming conditions with a compound of formula IV.

A further method of preparing a compound of the present invention in which $R_2$ is H, and n is 1, involves the Michael addition of a mercaptan of the formula $R_7SH$ with a compound of formula IX

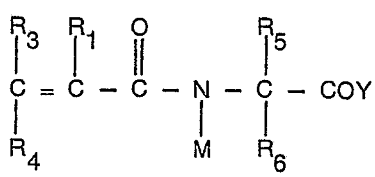

(IX)

In the foregoing reactions, it is preferred to employ compounds wherein the Y substituent, where present, is alkoxy, especially methoxy or ethoxy.

The aforesaid reactions can be conveniently carried out in organic solvents at temperatures ranging from 0° to the reflux temperature of the reaction mixture. The reaction periods are determined by the temperature selected for the reaction and the nature of the reactants. Progress of the reactions can be determined by removal of aliquots from the reaction mixture and analysis for either the reactants, the products, or both to determine the relative concentrations thereof. As is usual in organic reactions, even after substantially complete, reaction mixtures are digested by heating for extended periods usually at the reflux temperature of the reaction mixture.

Commonly, the solvent selected for the foregoing reactions is water-miscible in which case solvents such as dioxane, ethanol, methanol, acetone, dimethylformamide, diethylacetamide, and tetrahydrofuran are used. Water immiscible solvents can also be used.

In those reactions where hydrogen halide is formed, the reactions are preferably accomplished in the presence of a hydrogen halide acceptor such as inorganic bases, e.g. sodium hydroxide, sodium carbonate and lime, or organic bases such as amines, e.g., trimethylamine, aniline, pyridine and the like.

The amide forming conditions referred to herein involve the use of known derivatives of the described acids, such as the acyl halides, anhydrides, mixed anhydrides, lower alkyl esters, carbodiimides, carbonyl diimidazoles, and the like. The reactions are carried out in organic solvents such as acetonitrile, tetrahydrofuran, dioxane, acetic acid, methylene chloride, ethylene chloride and similar such solvents. The amide forming reaction will occur at room temperature or at elevated temperature. The use of elevated temperature is for convenience in that it permits somewhat shortened reaction periods. Temperatures ranging from 0°C. up to the reflux temperature of the reaction system can be used. As a further convenience, the amide forming reaction can be effected in the presence of a base such as tertiary organic amines, e.g., trimethylamine pyridine, picolines and the like, particularly where hydrogen halide is formed by the amide-forming reaction, e.g., acyl halide and amino compound. Of course, in those reactions where hydrogen halide is produced, any of commonly used hydrogen halide acceptors can also be used.

In the condensation of an alpha haloacid derivative of formula VI herein, similar reaction conditions, solvents and hydrogen halide acceptors can be used as for amide formation.

Various substituents on the present new compounds can be present in the starting compounds or added after formation of the amide products by the known methods of substitution or conversion reactions. Thus, the nitro group can be added to the final product by nitration of an aromatic ring and the nitro group converted to other groups, such as amino by reduction, and halo by diazotization of the amino groups and replacement of the diazo group. Other reactions can be effected on the formed amide product. Amino groups can be alkylated to form mono and dialkylamino groups, mercapto and hydroxy groups can be alkylated to form corresponding ethers. Thus, substitution or alteration reactions can be employed to provide a variety of substituents throughout the molecule of the final products. Of course, reactive groups where present should be protected by suitable blocking groups during any of the aforesaid reactions, particularly the condensation reactions to form the amide linkages.

The salts of the present new compounds, especially the pharmaceutically-acceptable salts, can be formed using standard procedures. Often, they are formed *in situ* during the preparation of the present new mercaptoamidoamino acids.

Thus, salts with alkali or alkaline earth metals can be formed by dissolution of the selected acid in a solution of the metal hydroxide in aqueous solution and the salt obtained by evaporation or precipitation. In this manner, the sodium, potassium and calcium salts can be prepared.

Salts with ammonia and amines can also be similarly prepared. A variety of amines can be used such as morpholine, piperidine, N-methylaniline, aminoethanols such as diethanolamine and triethanolamine, and similar such amines.

The present compounds obviously exist in stereoisomeric forms and the products obtained thus can be mixtures of the isomers, which can be resolved. Alternatively, by selection of specific isomers as starting compounds, the preferred stereoisomer can be produced. Therefore, the preferred forms, where each asymmetric center (chiral center) is S-configuration, are preferably prepared by the stereospecific route rather than attempting resolution of mixtures of isomers. The compoundss in which the S-configuration exists at all asymmetric centers are the most active; those in which the R-configurations exist are of intermediate activity.

The invention is further illustrated by the following examples.

### Example 1

N-[3-(3-Acetylthio-2-methylpropanoyl-thio)-2-methyl-propanoyl]-N-cyclopentylglycine-t-butyl ester

To a mixture of 3 g (0.01M) of N-(3-mercapto-2-methyl propanoyl)-N-cyclopentylglycine-t-butyl ester, 1.2 g (0.012M) triethylamine in 100 ml methylene chloride was added a solution of 1.8 g (0.01M) of 3-acetylthio-2-methyl propanoyl chloride and stirring continued for 8 hours. It was then washed with water, dried over $MgSO_4$ and evaporated to dryness leaving an oily residue which was purified by HPLC using ethyl acetate — hexane (1:3) as eluent.

### Example 2

N-[3-(3-Acetylthio-2-methylpropanoylthio)-2-methyl-propanoyl]-N-cyclopentylglycine

To a cold solution of 4.5 g (0.01) of N-[3-(3-acetylthio-2-methylpropanoylthio)-2-methyl-propanoyl]-N-cyclopentylglycine-t-butyl ester in 50 ml methylene chloride was added 2.2 g (0.011M) trimethylsilyl iodide in 10 ml methylene chloride and stirring continued for 2 hours. Ice was then added and extracted with 5% $NaHCO_3$. The basic solution was acidified with acetic acid, extracted with ethylacetate, dried over $MgSO_4$ and evaporated to dryness. The oily residue was purified by HPLC using HOAc—$ETOA_c$-hexane (4:46:50) as eluent.

### Example 3

N-(3-Phenylcarbamoylthio-2-methylpropanoyl)-N-cyclopentylglycine

A mixture of 4.9 g (0.02M) of N-(3-mercapto-2-methyl propanoyl)-N-cyclopentylglycine, 8 ml of phenyl isocyanate in 5 ml methylene chloride was stirred at room temperature for 72 hours. The mixture was

diluted with ether and filtered. Ether solution was washed with water, dried over $MgSO_4$ and evaporated to dryness. The crude residue was dissolved in acetonitrile, treated with charcoal and crystallized by addition of ether, m.p. 170—172°C.

Example 4

N-[3-(N'-Carbethoxymethylcarbamoylthio)-2-methyl propanoyl]-N-cyclopentylglycine-5-butyl ester

A mixture of 5 g of N-(3-mercapto-2-methyl propanoyl)-N-cyclopentylglycine-5-butyl ester and 6 ml of ethyl isocyanatoacetate was stirred at 80°C. for 48 hours. The mixture was cooled, stirred with water and filtered. The aqueous layer was extracted with ether. The ether extract was washed with water, dried over $MgSO_4$ and evaporated to dryness. The oily residue was purified by HPLC using EtOAC-hexane (3:17) as eluent.

Example 5

N-[3-(N'-Carbethoxymethylcarbamoylthio)-2-methyl-propanoyl]-N-cyclopentylglycine

To an ice cold solution of 2.9 g (7 mmol) of N-[3-(N'-carbethoxymethylcarbamoylthio)-2-methyl propanoyl]-N-cyclopentylglycine-t-butyl ester in 25 ml methylene chloride was slowly added 1 ml (7.5 mmol) of trimethylsilyl iodide in 5 ml methylene chloride and stirring continued for 2 more hours. Ice was added and extracted with 5% $NaHCO_3$ solution. The base extract was acidified with acetic acid and extracted with ethyl acetate which was dried over $MgSO_4$ and evaporated to dryness. The remaining oily residue 1.5 g was purified by HPLC using HOAC-EtoAC-hexane (4:46:50) as eluent.

Example 6

N-(3-Thioethylformatothio-2-methyl-propanoyl)-N-cyclopentylglycine-t-butyl ester

To a solution of 3 g (0.01) of N-(3-mercapto-2-methyl propanoyl)-N-cyclopentylglycine-t-butyl ester, 3 ml triethylamine in 50 ml ether was added 1.5 g (0.012) ethyl chlorothioformate in 10 ml ether, stirring continued for 16 hours. It was then washed with water, dried and evaporated leaving oily residue which crystallized on standing. Recrystallization from ether-hexane, m.p. 106—108°C.

Example 7

N-(3-Thioethylformatothio-2-methyl-propanoyl)-N-cyclopentylglycine

To a cold mixture of 2.6 g (0.007) of N-(3-thioethylformatothio-2-methyl propanoyl)-N-cyclopentyl-glycine-t-butyl ester in 50 ml $CH_2Cl_2$ was added a solution of 1 ml (0.0075) of trimethylsilyl iodide in 10 ml $CH_2Cl_2$, stirring continued for 2 hours. Ice was added, extracted with 5% $NaHCO_3$, acidified with HOAC, extracted with EtOAC, washed with $H_2O$, dried over $MgSO_4$ and evaporated to dryness. The oily residue was purified by HPLC using HOAC-hexane-EtOAC (3:48:50), m.p. 83—85°C.

Example 8

N-(3-Ethyloxalylthio-2-methylpropanoyl)-N-cyclopentylglycine-t-butyl ester

To a mixture of 9 g (0.03) of N-(3-mercapto-2-methyl propanoyl)-N-cyclopentylglycine-t-butyl ester, 5 ml $Et_3N$ in 150 ml ether was added 5 g (0.035) of ethyl chlorooxalate in 10 ml ether and stirred at room temperature for 6 hours. 200 ml ether was added, washed with water, 3% HCl, water saturated with $NaHCO_3$, water dried over $MgSO_4$, evaporated to dryness leaving an oily residue which was purified by HPLC using 20% EtOAC-hexane, m.p. 67—70°C.

Example 9

N-(3-Ethyloxalylthio-2-methylpropanoyl)-N-cyclopentylglycine

To a cold mixture of 3 g N-(3-ethyloxalylthio-2-methylpropanoyl)-N-cyclopentylglycine-t-butyl ester in 50 ml methylene chloride was added 1 ml of trimethyl silyl iodide in 10 ml methylene chloride. Stirring continued for 2 hours. Ice was then added, extracted with 5% $NaHCO_3$ solution, acidified with acetic acid and extracted with ethylacetate. The extract was washed with water, dried over $MgSO_4$ and evaporated to dryness. The oily residue was purified by HPLC using HOAC-EtOAC-hexane (4:46:50).

By following the procedures described above, the following additional compounds in which Y is hydroxy,

n is 1, $R_1$, $R_3$, $R_4$, $R_5$ and $R_6$ are hydrogen, $R_2$ is hydrogen or lower alkyl and M is cyclopentyl, can be prepared:

| $R_2$ | $R_7$ |
|---|---|
| $CH_3$ | furyl |
| $CH_3$ | thienyl |
| H | pyridyl |
| $CH_3$ | furfuryl |
| $CH_3$ | 2-thioketocyclopentyl |
| H | 2-thioketocyclohexyl |
| $CH_3$ | 2-imidazolyl |
| $CH_3$ | 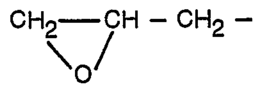 |
| $CH_3$ | 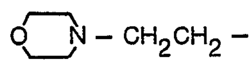 |
| H | 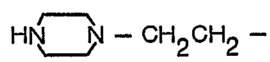 |
| $CH_3$ | 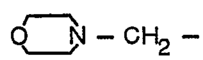 |
| $CH_3$ | 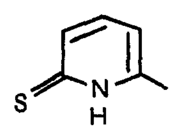 |

The compounds of the present invention have demonstrated potent activity (of the order of $I_{50}$ of 0.01 to 0.05 micromols) in the angiotensin converting enzyme (ACEI activity) when tested by the method described in Science *196,* 441—4 (1977). As such, these compounds would be very useful in the treatment of hypertension. The compounds may be administered orally or parenterally in the treatment of hypertension in animals, and it will be within the skill of the practitioner to determine the exact amount to be administered and the mode of administration.

**Claims**

1. Mercapto amino acids of the formula I

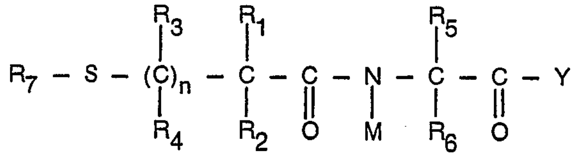

(I)

wherein

$R_1$, $R_2$, $R_3$, $R_4$, $R_5$, and $R_6$ are hydrogen, alkyl, alkenyl, alkynyl, aryl, aralkyl and cycloalkyl and may be the same or different,

n is an integer from 0 to 4 inclusive,

M is alkenyl, alkynyl, cycloalkyl, cycloalkylalkyl, polycycloalkyl, polycycloalkylalkyl, aryl, aralkyl, heterocyclic, heterocyclicalkyl, fused arylcycloalkyl, fused arylcycloalkylalkyl, fused heterocycliccycloalkyl, fused heterocycliccycloalkylalkyl, alkoxyalkyl, alkylmercapto, alkylaminoalkyl or dialkylaminoalkyl,

Y is hydroxy, alkoxy, amino, or substituted amino, alkanoylamino, aryloxy, aminoalkoxy or hydroxyalkoxy, and

$R_7$ is cycloalkyl, cycloalkylalkyl, heterocyclic, heterocyclicalkyl,

—CS—SR, 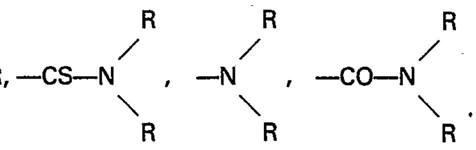

—$CH_2CO_2R$, —CHM—$CO_2R$, —$CH_2$—CO—SR, —CO—NH—$CH_2CO_2R$

or 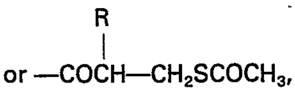, —CO—$CO_2R$,

wherein R is hydrogen, alkyl or aryl,
and salts thereof, especially pharmaceutically acceptable salts such as the alkali metal, alkaline earth metal, and amine salts, with the proviso that when $R_7$ is

—$CH_2CO_2R$, 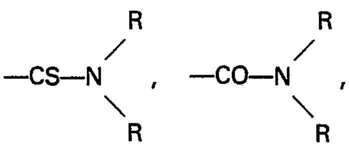

—CO—$CO_2R$, —CS—SR or cycloalkyl, M is not aryl.

2. Mercapto amino acids of the formula

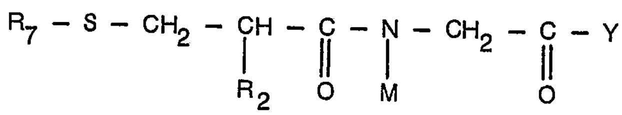

wherein

Y is hydroxy or lower alkoxy,

$R_2$ is hydrogen or lower alkyl,

M is a cycloalkyl having 3 to 7 carbon atoms, and

$R_7$ is —$COCO_2R$, —$CON(R)_2$, —$CH_2CO_2R$, or —$COCH(CH_3)$—$CH_2SCOCH_3$, wherein R is hydrogen, lower alkyl or phenyl.

3. A compound according to claim 2 wherein M is cyclopentyl.

4. A compound according to any of claims 1 to 3 wherein Y is hydroxy.

5. A compound according to any of claims 1 to 4 wherein $R_7$ is —$CH_2CO_2H$.

6. A compound according to any of claims 1 to 4 wherein $R_7$ is —$CONHC_6H_5$.

7. A compound according to any of claims 1 to 4 wherein $R_7$ is —$COCH(CH_3)CH_2SCOCH_3$.

8. A compound according to any of claims 1 to 4 wherein $R_7$ is —$COCO_2C_2H_5$.

9. A pharmaceutical composition comprising at least one compound according to any of claims 1 to 8 as active ingredient together with pharmaceutically acceptable carriers and/or diluents.

10. A process of producing a compound of formula I which comprises
replacement of the hydrogen of the —SH group of a compound of formula II

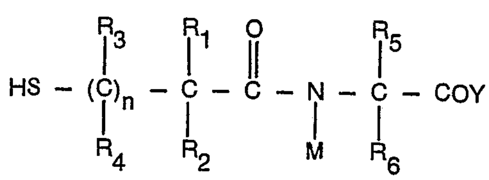

(II)

with a substituent representative of $R_7$;
or by reaction of a compound of formula III

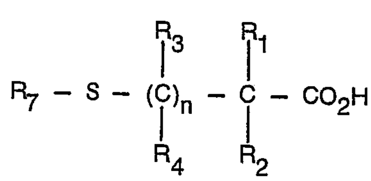

(III)

under amide forming conditions with a compound of formula IV

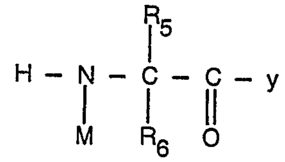

(IV)

or by reaction of a compound of formula V

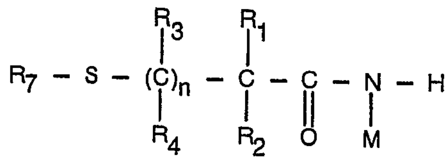

(V)

with an alpha haloacid of formula VI

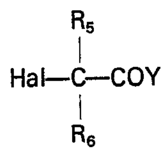

(VI)

or by Michael addition of a mercaptan of the formula

$R_7SH$

with a compound of formula IX

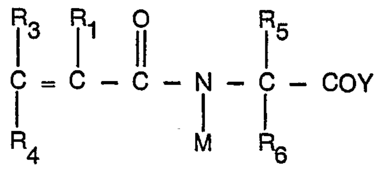

(IX)

$R_1$, $R_2$, $R_3$, $R_4$, $R_5$, $R_6$, n, M and Y in the formulas being defined as in claim 1, and optionally introducing substituents onto said molecules by substitution or alteration reactions; and optionally forming pharmaceutically-acceptable salts of the said products in which acid groups are present by reaction with suitable bases.

11. Process as in claim 10 wherein Y is alkoxy, preferably methoxy, ethoxy or hydroxy.

12. Process as in claim 10 or 11 wherein $R_2$ is H or lower alkyl;
$R_1$, $R_3$, $R_4$, $R_5$ and $R_6$ are H;
n is 1; and
$R_7$ is $—COCO_2R$, $—CON(R_2)$, $—CH_2CO_2R$ or $—COCH(CH_3)CH_2SCOCH_3$ wherein R is H, alkyl or phenyl;
M is cycloalkyl preferably containing 3 to 7 carbon atoms.

13. Process as in any of claims 10 and 12 wherein M is cyclopentyl.

14. Process as in any of claims 10 to 13 wherein $R_7$ is $—CH_2CO_2H$.

15. Process as in any of claims 10 to 13 wherein $R_7$ is $—CONHC_6H_5$.

16. Process as in any of claims 10 to 13 wherein $R_7$ is $—COCH(CH_3)CH_2SCOCH_3$.

17. Process as in any of claims 10 to 13 wherein $R_7$ is $—COCO_2C_2H_5$.

**Patentansprüche**

1. Mercaptoaminosäuren der Formel I

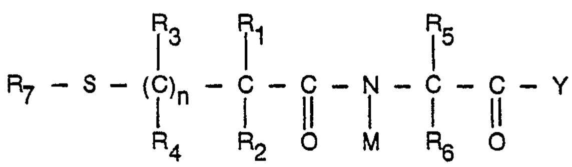

(I)

worin

$R_1$, $R_2$, $R_3$, $R_4$, $R_5$ und $R_6$ Wasserstoff, Alkyl, Alkenyl, Alkynyl, Aryl, Aralkyl und Cycloalkyl bedeuten und gleich oder verschieden sein können,

n eine ganze Zahl von 0 bis 4 ist,

M Alkenyl, Alkynyl, Cycloalkyl, Cycloalkylalkyl, Polycycloalkyl, Polycycloalkylalkyl, Aryl, Aralkyl, einen Heterocyclus, ein heterocyclisches Alkyl, ein kondensiertes Arylcycloalkyl, ein kondensiertes Arylcycloalkylalkyl, ein kondensiertes heterocyclisches Cycloalkyl, ein kondensiertes heterocyclisches Cycloalkylalkyl, Alkoxyalkyl, Alkylmercapto, Alkylaminoalkyl oder Dialkylaminoalkyl bedeutet,

Y Hydroxyl, Alkoxy, Amino oder substituiertes Amino, Alkanoylamino, Aryloxy, Aminoalkoxy oder Hydroxyalkoxy bedeutet, und

$R_7$ Cycloalkyl, Cycloalkylalkyl, einen Heterocyclus, ein heterocyclisches Alkyl,

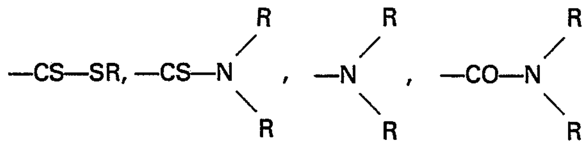

$—CH_2CO_2R$, $—CHM—CO_2R$, $—CH_2—CO—SR$, $—CO—NH—CH_2CO_2R$

oder 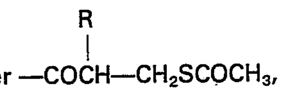, $—CO—CO_2R$, bedeutet,

worin

R Wasserstoff, Alkyl oder Aryl ist,

und Salze davon, insbesondere pharmazeutisch annehmbare Salze, wie die Alkalimetall-, Erdalkalimetall- und Aminsalze,

mit der Maßgabe, daß, wenn $R_7$

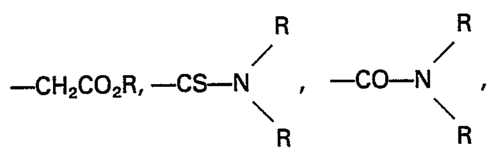

$—CO—CO_2R$, $—CS—SR$ oder Cycloalkyl ist, M nicht Aryl ist.

2. Mercaptoaminosäuren der Formel

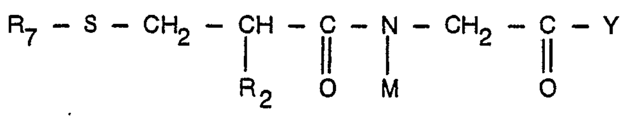

worin

Y Hydroxyl oder Niedrigalkoxy bedeutet,

$R_2$ Wasserstoff oder Niedrigalkyl bedeutet,

M ein Cycloalkyl mit 3 bis 7 Kohlenstoffatomen bedeutet und

$R_7$ $—COCO_2R$, $—CON(R)_2$, $—CH_2CO_2R$, oder $—COCH(CH_3)—CH_2SCOCH_3$ bedeutet,

worin

R Wasserstoff, Niedrigalkyl oder Phenyl ist.

3. Verbindung nach Anspruch 2, worin M Cyclopentyl ist.

4. Verbindung nach einem der Ansprüche 1 bis 3, worin Y Hydroxyl ist.

5. Verbindung nach einem der Ansprüche 1 bis bis 4, worin $R_7$ $—CH_2CO_2H$ ist.

6. Verbindung nach einem der Ansprüche 1 bis 4, worin $R_7$ $—CONHC_6H_5$ ist.

7. Verbindung nach einem der Ansprüche 1 bis 4, worin $R_7$ $—COCH(CH_3)CH_2SCOCH_3$ ist.

8. Verbindung nach einem der Ansprüche 1 bis 4, worin $R_7$ $—COCO_2C_2H_5$ ist.

9. Pharmazeutische Zusammensetzung, umfassend wenigstens eine Verbindung nach einem der Ansprüche 1 bis 8 als aktiven Bestandteil zusammen mit pharmazeutisch annehmbaren Trägern und/oder Verdünnungsmitteln.

10. Verfahren zur Herstellung einer Verbindung der Formel I, welches das Ersetzen des Waserstoff der —SH-Gruppe einer Verbindung der Formel II

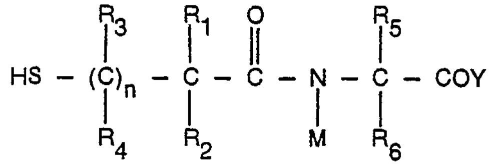

(II)

durch einen Substituenten in der Bedeutung von $R_7$, oder die Reaktion einer Verbindung der Formel III

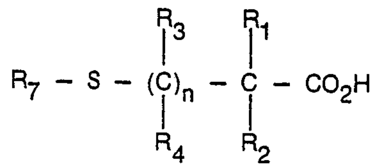

(III)

unter amidbildenden Bedingungen mit einer Verbindung der Formel IV

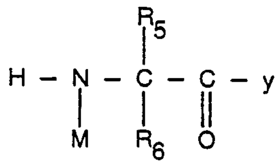

(IV)

oder die Reaktion einer Verbindung der Formel V

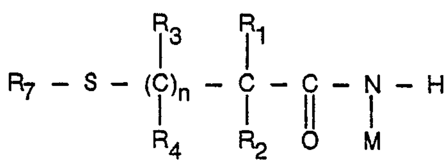

(V)

mit einer α-Halogensäure der Formel VI

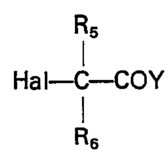

(VI)

oder die Michael-Addition eines Mercaptans der Formel

$R_7SH$

mit einer Verbindung der Formel IX

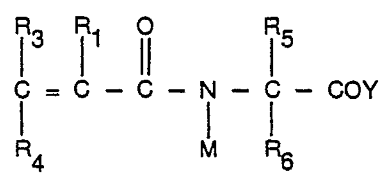

(IX)

worin $R_1$, $R_2$, $R_3$, $R_4$, $R_5$, $R_6$, n, M und Y in den Formeln die in Anspruch 1 angegebene Bedeutung besitzen, und

gegebenenfalls Substituenten in die Molekülen durch Substitutions- oder Änderungsreaktionen eingeführt werden und gegebenenfalls pharmazeutisch annehmbare Salze der Produkte, in denen Säuregruppen vorliegen, durch Reaktion mit geeigneten Basen gebildet werden, umfaßt.

11. Verfahren nach Anspruch 10, worin Y Alkoxy, vorzugsweise Methoxy, Ethoxy oder Hydroxy, ist.

12. Verfahren nach Anspruch 10 oder 11, worin

$R_2$ H oder Niedrigalkyl ist,

$R_1$, $R_3$, $R_4$, $R_5$ und $R_6$ H sind,

n 1 is und

$R_7$ —$COCO_2R$, —$CON(R_2)$, —$CH_2CO_2R$ oder —$COCH(CH_3)CH_2SCOCH_3$ ist,

worin R H, Alkyl oder Phenyl ist,

M Cycloalkyl, vorzugsweise mit 3 bis 7 Kohlenstoffatomen ist.

13. Verfahren nach einem der Ansprüche 10 und 12, worin M Cyclopentyl ist.

14. Verfahren nach einem der Ansprüche 10 bis 13, worin $R_7$—$CH_2CO_2H$ ist.

15. Verfahren nach einem der Ansprüche 10 bis 13, worin $R_7$ —$CONHC_6H_5$ ist.

16. Verfahren nach einem der Ansprüche 10 bis 13, worin $R_7$ —$COCH(CH_3)CH_2SCOCH_3$ ist.

17. Verfahren nach einem der Ansprüche 10 bis 13, worin $R_7$ —$COCO_2C_2H_5$ ist.

**Revendications**

1. Mercapto amino acides représentés par la formule I:

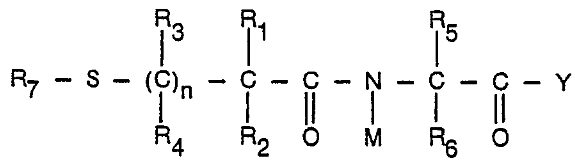

(I)

formule dans laquele:

$R_1$, $R_2$, $R_3$, $R_4$, $R_5$ et $R_6$ représentent hydrogène, alkyle, alcényle, alcynyle, aryle, aralkyle et cycloalkyle, et peuvent être identiques ou différents;

n est un nombre entier allant de 0 à 4 compris;

M représente alcényle, alcynyle, cycloalkyle, cycloalkylalkyle, polycycloalkyle, polycycloalkylalkyle, aryle, aralkyle, hétérocyclique, hétérocyclique-alkyle, arylcycloalkyle condensé, arylcycloalkylalkyle condensé, hétérocyclique-cycloalkyle condensé, hétérocyclique-cycloalkylalkyle condensé, alcoxyalkyle, alkylmercapto, alkylaminoalkyle ou dialkylaminoalkyle;

Y représente hydroxy, alcoxy, amino ou amino substitué, alcanoylamino, aryloxy, aminoalcoxy ou hydroxyalcoxy; et

$R_7$ représente cycloalkyle, cycloalkylalkyle, hététrocyclique, hétérocyclique-alkyle,

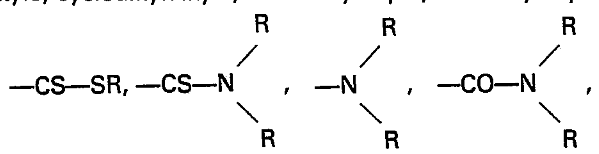

$-CH_2CO_2R$, $-CHM-CO_2R$, $-CH_2-CO-SR$, $-CO-NH-CH_2CO_2R$

ou 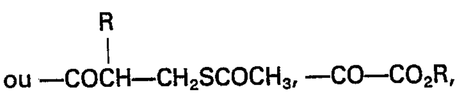

où R représente hydrogène, alkyle ou aryle, et leurs sels, en particulier, leurs sels pharmaceutiquement acceptables, tels que les sels de métaux alcalins, de métaux alcalino-terreux et d'amines, à la condition que, lorsque $R_7$ représente

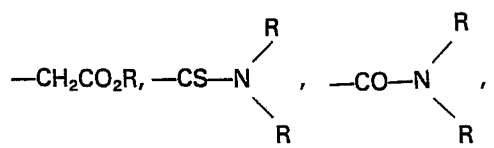

$-CO-CO_2R$, $-CS-SR$ ou cyclo-alkyle,

M ne représente pas aryle.

2. Mercapto amino acides représentés par la formule:

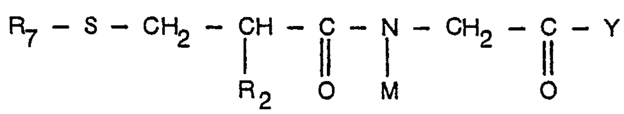

dans laquelle:

Y représente hydroxy ou alcoxy inférieur;

$R_2$ représente hydrogène ou alkyle inférieur;

M représente un groupe cycloalkyle renfermant 3 à 7 atomes de carbone; et

$R_7$ représente $-COCO_2R$, $-CON(R)_2$, $-CH_2CO_2R$, ou $-COCH(CH_3)-CH_2SCOCH_3$, où R représente hydrogène, alkyle inférieur ou phényle.

3. Composé selon la revendication 2, dans lequel M représente cyclopentyle.

4. Composé selon l'une des revendications 1 à 3, dans lequel Y représente hydroxy.

5. Composé selon l'une des revendications 1 à 4, dans lequel $R_7$ représente $-CH_2CO_2H$.

6. Composé selon l'une des revendications 1 à 4, dans lequel $R_7$ représente $-CONHC_6H_5$.

7. Composé selon l'une des revendications 1 à 4, dans lequel $R_7$ représente $-COCH(CH_3)CH_2SCOCH_3$.

8. Composé selon l'une des revendications 1 à 4, dans lequel $R_7$ représente $-COCO_2C_2H_5$.

9. Composition pharmaceutique comprenant au moins un composé selon l'une des revendications 1 à 8 comme ingrédient actif, conjointement avec des supports et/ou diluants pharmaceutiquement acceptables.

10. Procédé d'obtention d'un compose de formule I, qui consiste à remplacer l'hydrogène du groupe —SH d'un composé de formule II:

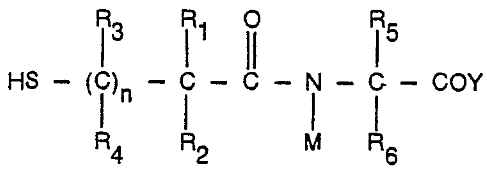 (II)

par un substituant représentatif de $R_7$;
ou à faire réagir un composé de formule III:

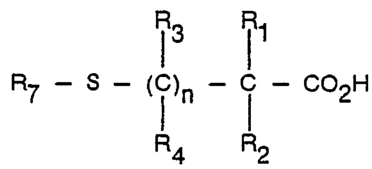 (III)

dans des conditions de formation d'un amide, avec un composé de formule IV:

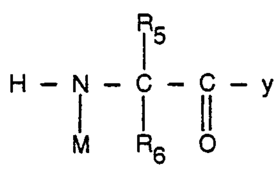 (IV)

ou à faire réagir un composé de formule V:

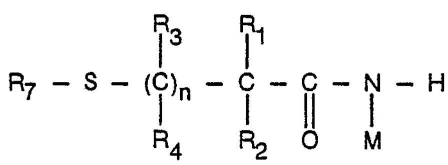 (V)

avec un alpha haloacide de formule VI:

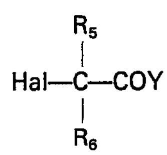 (VI)

ou à effectuer une addition de Michael d'un mercaptan de formule:

$R_7SH$

avec un composé de formule IX:

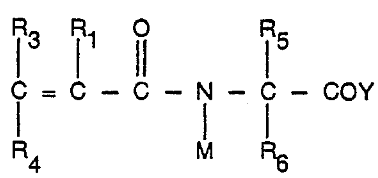 (IX)

$R_1$, $R_2$, $R_3$, $R_4$, $R_5$, $R_6$, n, M et Y dans les formules étant tels que définis à la revendication 1, et, de façon facultative, à introduire des substituants sur lesdites molécules par des réactions de substitution ou de modification; et, de façon facultative, à former des sels pharmaceutiqement acceptables desdits produits, dans lesquels des groupes acides sont présents, par réaction avec des bases appropriées.

11. Procédé selon la revendication 10, dans lequel Y représente un groupe alcoxy, de préférence, méthoxy, éthoxy ou hydroxy.

12. Procédé selon la revendication 10 ou 11, dans lequel:
$R_2$ représente H ou alkyle inférieur;
$R_1$, $R_3$, $R_4$, $R_5$ et $R_6$ représentent H;
n vaut 1; et

$R_7$ représente $-COCO_2R$, $-CON(R)_2$, $-CH_2CO_2R$ ou $-COCH(CH_3)CH_2SCOCH_3$, où R représente H, alkyle ou phényle.

M représente un reste cycloalkyle contenant, de préférence, 3 à 7 atomes de carbone.

13. Procédé selon l'une des revendications 10 et 12, dans lequel M représente cyclopentyle.

14. Procédé selon l'une des revendications 10 à 13, dans lequel $R_7$ représente $-CH_2CO_2H$.

15. Procédé selon l'une des revendications 10 à 13, dans lequel $R_7$ représente $-CONHC_6H_5$.

16. Procédé selon l'une des revendications 10 à 13, dans lequel $R_7$ représente

$$-COCH(CH_3)CH_2SCOCH_3.$$

17. Procédé selon l'une des revendications 10 à 13, dans lequel $R_7$ représente $-COCO_2C_2H_5$.